# EUROPEAN PATENT SPECIFICATION

(11) **EP 2 027 839 B1**
(45) Date of publication and mention of the grant of the patent: **11.04.2012**
(21) Application number: 08161041.2
(22) Date of filing: 24.07.2008
(51) Int. Cl.: A61F 13/14

(54) **Milk absorbent cup-shaped breast pad provided with antibacterical product, and method for its formation**
Saugfähige körbchenförmige Stilleinlage mit antibakteriellem Produkt sowie Herstellungsverfahren dafür
Coussinet de poitrine en forme de bonnet absorbant le lait doté d'un produit antibactérien et son procédé de formation

(30) Priority: 26.07.2007 IT MI20071505
(43) Date of publication of application: 25.02.2009
(73) Proprietor: Artsana S.p.A., 22070 Grandate (CO) (IT)
(72) Inventor: Lo Piccolo, Francesco, 90141, Palermo (IT); Montepara, Paola, 66036, Orsogna (Chieti) (IT)
(74) Representative: Ripamonti, Enrico

(56) References cited:
- WO-A1-96/41601
- WO-A2-2006/130026
- US-A- 4 692 374
- US-A1- 2003 105 445
- US-A1- 2003 220 048
- US-A1- 2006 052 033
- US-H1- H2 062

## Description

The present invention relates to a milk absorbent cup-shaped breast pad, in accordance with the introduction to the main claim, so as disclosed in US-2003105445.

During lactation, mothers are subject to irritation, rhagades and milk leakage. New mothers therefore frequently use milk absorbent breast pads.

Currently available milk absorbent breast pads are designed to absorb milk leakages and avoid skin maceration, with the object of preventing irritation. Irritation and rhagades can however still appear by the pulling effect on the nipple exerted by the child. In these cases, currently available breast pads do not provide effective breast protection against bacteria, microbes and funguses, hence do not create hygienic conditions ideal for healing.

It is also known to use antibacterial or antimicrobic products in fabrics for producing children's clothing, handkerchiefs or the like.

Methods for forming these products are described for example in US4282366, US4504541, US4615937, US4408996 and US4414268. However these patents do not describe methods for forming milk absorbent breast pads able to solve the aforesaid problems.

An object of the present invention is to provide a milk absorbent cup-shaped breast pad presenting an antibacterial characteristic enabling the breast surface to be preserved from bacterial proliferation, so preventing irritation and rhagades and maintaining the breast under ideal conditions for facilitating healing thereof.

Another object is to offer a method for forming such a milk absorbent breast pad.

These and other objects which will be apparent to the expert of the art are attained by a milk absorbent cup-shaped breast pad and a method for its formation in accordance with the accompanying claims.

The present invention will be more apparent from the accompanying drawing, which is provided by way of non-limiting example and in which:
Figure 1 is a view of a breast pad of the invention seen from above;
Figure 2 is a section on the line 2-2 of Figure 1; and
Figure 3 is a side view of the breast pad of Figure 1.

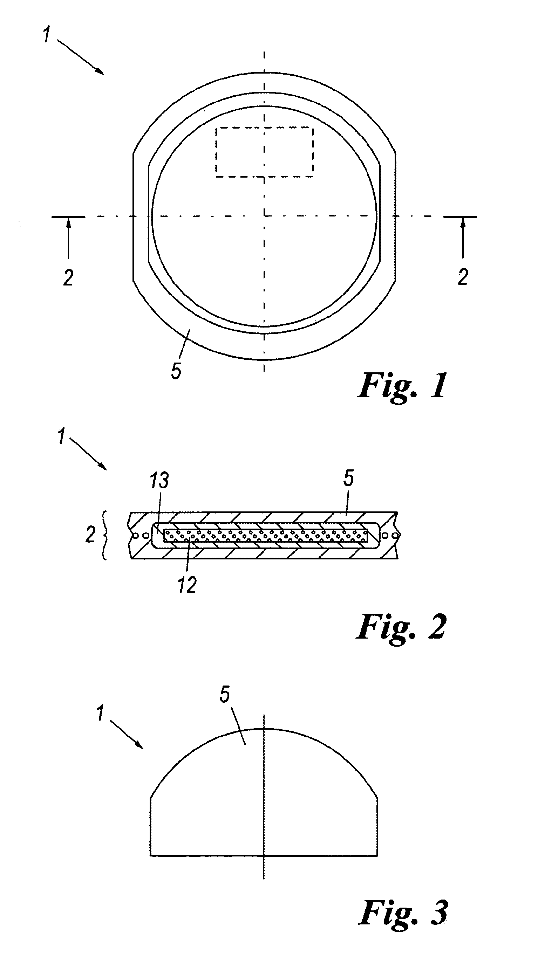

With reference to said figures, a breast pad of the invention is indicated overall by 1 and comprises a layered body 2. The breast pad has a dished shape, as visible in Figure 3.

According to the invention, the breast pad 1 comprises an outer covering layer 5 of polyolefinic bicomponent fibre treated according to the invention with an antibacterial or antimicrobic product of "attractive" type (attracting bacteria) such as the product AEGIS AEM 5772/5 or similar (such as a product described for example in US4408996 or US4504541). More particularly, this fibre can be composed of polypropylene and/or polyethylene; after its weaving (to hence be able to define the layer 5), the antibacterial product is spray-applied to the fabric. The quantity of this product is chosen between 0.5% and 20% of the fibre weight, preferably between 1% and 4%, and advantageously between 1.8% and 2.2%, this latter value providing suitable homogeneity of fibre treatment such as to allow correct antibacterial or antimicrobic product presence in the entire layer 5 and correct protection of the mother's breast when the breast pad is in contact therewith.

"Coupling" between the fibre and antibacterial product is preferably obtained during the fibre production stage: the antibacterial product is mixed with the plastic used to define the fibre at about 60°C and is also sprayed onto it after spinning. This product is then fixed permanently to the fibre, so that none is released onto the mother's skin even if the layer 5 is in direct contact with the skin. This fixing is advantageously achieved by thermally stabilizing the fibre with the antibacterial product at 110°C for 6 minutes.

The breast pad also internally comprises a layer 12 of soft absorbent material (for example of cellulose mixed with super-absorbent polymers) covered or wrapped with a thin absorbent layer 13 of cellulose tissue or other material; the breast pad also comprises elastic inserts allowing optimal breast conformation (or wearability).

In the milk absorbent breast pad obtained in this manner, the antibacterial activity is developed within the cup-shaped breast pad itself, there being no transfer of the antibacterial molecule onto the skin.

## Claims

1. A milk absorbent cup-shaped breast pad comprising a multi-layered body (2), said body comprising absorbent inner layers (12, 13) and an outer layer (5) to be placed in contact with the skin of a mother's breast, said layer being of woven fibre, said outer layer (5) being treated with an antibacterial or antimicrobic product, said product being present in a percentage between 0.5% and 20% of the weight of the fibre defining said layer, said antibacterial or antimicrobic product is sprayed onto the fabric defining the outer layer (5), the fibre being a bicomponent fibre of polyolefinic nature, such as polypropylene and polyethylene, **characterised in that** said antibacterial or antimicrobic product is mixed with the polyolefinic plastic before its transformation into fibre.

2. A milk absorbent breast pad as claimed in claim 1, **characterised in that** said antibacterial or antimicrobic product is present in a percentage between 1% and 4% of the weight of the fibre, preferably between 1.8% and 2.2% of the weight of this latter.

3. A milk absorbent breast pad as claimed in claim 1, **characterised in that** said antibacterial or antimicrobic product is AEGIS AEM 5772/5.

4. A method for forming a multi-layered cup-shaped breast pad (1), said breast pad comprising absorbent inner layers (12, 13) and an outer layer (5) to be placed in contact with the skin of a mother's breast, said layer being of woven fibre, **characterised by** associating securely with the outer layer (5) an antibacterial or antimicrobic product, said product being in a percentage between 0.5% and 20% of the weight of the fibre of said layer, said product being sprayed onto the layer (5), **characterised in that** said layer is of bicomponent fibre of polyolefinic nature, said product being mixed with the plastic before its transformation into fibre.

5. A method as claimed in claim 4, **characterised in that** said mixing is carried out at 60°C.

6. A method as claimed in claim 4, **characterised in that** the fibre with antibacterial or antimicrobic product is temperature-stabilized, preferably at 110°C, for a predetermined time period, advantageously 6 minutes.

7. A method as claimed in claim 4, **characterised in that** the percentage of antibacterial or antimicrobic product is chosen between 1% and 4% of the weight of the fibre, advantageously between 1.8% and 2.2% of the weight of this latter.

## Patentansprüche

1. Ein Milch absorbierendes, schalenförmiges Brustkissen, das einen vielschichtigen Körper (2) umfasst, wobei der genannte Körper jeweils absorbierende innere Schichten (12, 13) sowie eine äußere Schicht (5) umfasst, die in Berührung mit der Haut der Mutterbrust angeordnet werden soll, wobei die genannte Schicht aus einer Gewebefaser besteht, und die genannte äußere Schicht (5) jeweils mit einem antibakteriellen oder antimikrobiellen Produkt behandelt wird, und das genannte Produkt jeweils in einem Prozentsatz zwischen 0,5% und 20% des Gewichts der Faser vorhanden ist, aus der die genannte Schicht besteht, wobei das genannte antibakterielle oder antimikrobielle Produkt auf das Gewebe aufgesprüht wird, aus dem die äußere Schicht (5) besteht, und die Faser eine Polyolefin-Zweikomponentenfaser wie Polypropylen und Polyäthylen ist, **dadurch gekennzeichnet, dass** das genannte antibakterielle oder antimikrobielle Produkt vor seiner Umwandlung in Fasern jeweils mit dem Polyolefin-Kunststoff vermischt wird.

2. Ein Milch absorbierendes Brustkissen gemäß Anspruch 1, **dadurch gekennzeichnet, dass** das genannte antibakterielle oder antimikrobielle Produkt jeweils in einem Prozentsatz zwischen 1% und 4% des Gewichts der Faser vorhanden ist, und zwar vorzugsweise zwischen 1,8% und 2,2% des Gewichts von dieser Letzteren.

3. Ein Milch absorbierendes Brustkissen gemäß Anspruch 1, **dadurch gekennzeichnet, dass** es sich bei dem genannten antibakteriellen oder antimikrobillen Produkt um AEGIS AEM 5772/5 handelt.

4. Eine Methode zur Bildung eines vielschichtigen, schalenförmigen Brustkissens (1), wobei das genannte Brustkissen jeweils absorbierende innere Schichten (12, 13) und eine äußere Schicht (5) umfasst, die in Berührung mit der Haut der Mutterbrust angeordnet werden soll, wobei die genannte Schicht aus einer Gewebefaser besteht, **dadurch gekennzeichnet, dass** mit der äußeren Schicht (5) ein antibakterielles oder antimikrobielles Produkt verbunden wird, wobei das genannte Produkt jeweils in einem Prozentsatz zwischen 0,5% und 20% des Gewichts der Faser der genannten Schicht vorhanden ist und das genannte Produkt auf die Schicht (5) aufgesprüht wird, **dadurch gekennzeichnet, dass** die genannte Schicht aus einer Polyolefin-Zweikompomponentenfaser Faser besteht, wobei das genannte Produkt vor seiner Umwandlung in Fasern jeweils mit dem Kunststoff vermischt wird.

5. Eine Methode gemäß Anspruch 4, **dadurch gekennzeichnet, dass** die genannte Mischung bei 60°C erfolgt.

6. Eine Methode gemäß Anspruch 4, **dadurch gekennzeichnet, dass** die Faser mit dem antibakteriellem oder antimikrobiellem Produkt während einer vorbestimmmten Zeit temperaturstabilisiert wird, und zwar vorzugsweise bei 110°C und vorteilhafterweise 6 Minuten lang.

7. Eine Methode gemäß Anspruch 4, **dadurch gekennzeichnet, dass** der Prozentsatz des antibakteriellen oder antimikrobiellen Produtks jeweils zwischen 1% und 4% des Gewichts der Faser gewählt wird, und zwar vorteilhafterweise zwischen 1,8% und 2,2% des Gewichts dieser Letzteren.

## Revendications

1. Coussinet d'allaitement en forme de coupelle recueille lait comprenant un corps multicouches (2), ledit corps comprenant des couches internes absorbantes (12, 13) et une couche extérieure (5) devant être placée au contact de la peau du sein d'une mère, ladite couche étant réalisée en fibre tissée, ladite couche extérieure (5) étant traitée avec un produit antimicrobien ou antibactérien, ledit produit étant présent dans un pourcentage compris entre 0,5 % et 20 % du poids de la fibre définissant ladite couche, ledit produit antimicrobien ou antibactérien étant projeté sur le tissu définissant la couche extérieure (5), la fibre étant une fibre bi-composants de nature polyoléfine, telle que le polypropylène et le polyéthylène, **caractérisé en ce que** ledit produit antimicrobien ou antibactérien est mélangé au plastique polyoléfine avant sa transformation en fibre.

2. Coussinet d'allaitement recueille lait selon la revendication 1, **caractérisé en ce que** ledit produit antimicrobien ou antibactérien est présent dans un pourcentage compris entre 1 % et 4 % du poids de la fibre, de préférence entre 1,8 % et 2,2 % du poids de cette dernière.

3. Coussinet d'allaitement recueille lait selon la revendication 1, **caractérisé en ce que** le produit antimicrobien ou antibactérien est AEGIS AEM 5772/5.

4. Méthode pour former un coussinet d'allaitement multicouches en forme de coupelle (1), ledit coussinet d'allaitement comprenant des couches internes absorbantes (12, 13) et une couche extérieure (5) devant être placée au contact de la peau du sein d'une mère, ladite couche étant réalisée en fibre tissée, **caractérisée en ce que** l'on associe fixement un produit antimicrobien ou antibactérien à la couche extérieure (5), ledit produit étant dans un pourcentage compris entre 0,5 % et 20 % du poids de la fibre de ladite couche, ledit produit étant projeté sur la couche (5), **caractérisée en ce que** ladite couche est en fibre bi-composants de nature polyoléfine, ledit produit étant mélangé au plastique avant sa transformation en fibre.

5. Méthode selon la revendication 4, **caractérisée en ce que** ledit mélange est réalisé à 60°C.

6. Méthode selon la revendication 4, **caractérisée en ce que** la fibre avec du produit antimicrobien ou antibactérien est stabilisée thermiquement, de préférence à 110°C, pendant une période de temps préétablie, avantageusement 6 minutes.

7. Méthode selon la revendication 4, **caractérisée en ce que** le pourcentage de produit antimicrobien ou antibactérien est choisi entre 1 % et 4 % du poids de la fibre, avantageusement entre 1,8 % et 2,2 % du poids de cette dernière.
